# FASCICULE DE BREVET EUROPEEN

(11) **EP 3 834 860 B1**
(45) Date de publication et mention de la délivrance du brevet: **14.08.2024**
(21) Numéro de dépôt: 19215310.4
(22) Date de dépôt: 11.12.2019
(51) Int. Cl.: A61M 1/36, A61K 38/16, A61K 38/49

(54) **DISPOSITIF DE CIRCULATION EXTRACORPORELLE DU SANG**
VORRICHTUNG FÜR DIE EXTRAKORPORALE BLUTZIRKULATION
DEVICE FOR EXTRACORPOREAL CIRCULATION OF BLOOD

(43) Date de publication de la demande: 16.06.2021
(73) Titulaire: Infomed SA, 1252 Meinier (CH)
(72) Inventeur: Favre, Olivier, 1226 Thônex (CH)
(74) Mandataire: Micheli & Cie SA

(56) Documents cités:
- EP-A1- 1 430 920
- WO-A1-2007/062197
- WO-A1-94/21124
- US-A1- 2012 273 415

## Description

La présente invention se rapporte à un dispositif de circulation extracorporelle du sang possédant une boucle de circulation extracorporelle de sang.

Des dispositifs réalisant une circulation extracorporelle de sang existent et sont largement décrits dans la littérature ; ils assurent de nombreuses fonctions par exemple pour extraire des éléments du sang (globules blancs, plasma, plaquettes, ... ci-après désigné par « aphérèse »), pour soigner des patients présentant une pathologie nécessitant une épuration du sang telle que la défaillance rénale (hémodialyse, hémofiltration, hémodiafiltration, ci-après désigné par « dialyse »), pour assurer la circulation du sang lors d'une chirurgie cardiaque (ci-après désigné par « CEC ») ou afin d'oxygéner le sang (ci-après désigné par « ECMO »). La dialyse, les CEC et l'ECMO sont toujours à buts thérapeutiques, c'est-à-dire destinés à soigner des patients alors que l'aphérèse s'adresse soit à des patients, atteints par exemple de maladie auto-immune ou d'hypercholestérolémie, soit à des donneurs qui sont des personnes saines qui font don d'éléments de leur sang comme du plasma, des plaquettes ou des globules rouges. Il existe pour cela différents systèmes de circulation du sang qui comprennent tous des tubes pour faire circuler le sang et des pompes qui créent le flux, comme par exemple des pompes péristaltiques, des pompes centrifuges ou des pompes à membrane. Les différents moyens connus pour faire circuler le sang à l'extérieur du patient en formant une boucle de circulation extracorporelle (ci-après désignée par « boucle ») sont décrits dans la littérature depuis plus de 40 ans ; ils ont tous en commun d'activer la coagulation du sang via deux mécanismes principaux que sont le contact du sang avec des substances telles que le plastique ou l'air et les flux turbulents ou à pression variables qui résultent de la configuration des circuits.

La coagulation du sang est réalisée par une cascade d'événements dont beaucoup sont encore méconnus et qui peut se résumer de manière simple comme suit : les plaquettes présentes dans le sang sont d'abord activées, c'est-à-dire mises dans un état qui les rend promptes à se lier, par une situation inhabituelle comme le contact avec un matériau inconnu ou une variation forte et brutale de pression. Elles s'agglutinent alors pour former un « clou plaquettaire » typiquement fait pour boucher une plaie comme dans le cas d'une coupure de la peau. Ce clou est ensuite renforcé par différents facteurs de la coagulation, notamment le fibrinogène, pour devenir un « clou de fibrine ». Si ce phénomène est souhaité lorsqu'une plaie saigne afin d'arrêter l'hémorragie, il est à éviter dans les boucles de circulation du sang puisqu'il réduit la capacité de la boucle à faire circuler le sang avant de l'empêcher complètement rendant ainsi ladite boucle inutilisable.

La cascade de coagulation interagit avec celle de l'inflammation et celle de l'immunité qui sont elles aussi souvent modifiées pendant la période de circulation dans la boucle soit directement par le traitement réalisé, par exemple lorsque celui-ci retire des médiateurs de l'inflammation ou des anticorps, ou du fait de facteurs externes comme par exemple l'utilisation d'antibiotiques ; les paramètres de la cascade de coagulation sont donc susceptibles d'être modifiés non seulement par le passage du sang dans la boucle mais aussi du fait de la situation du patient sur la durée de la circulation, laquelle peut être de plusieurs jours voire semaines. De nombreux phénomènes agissent ainsi pour coaguler le sang, ou au contraire ne pas le laisser coaguler, et il est illusoire de penser tous les connaitre de façon à prévenir toute coagulation dans une boucle. Ainsi malgré tous les moyens mis en oeuvre à ce jour, la coagulation du sang dans les boucles de circulation extracorporelle demeure une des raison principales de changement de boucles non désiré, ce qui se fait en arrêtant l'appareil après avoir si possible retourné son sang au patient/donneur puis en mettant un circuit neuf et en préparant l'appareil avec les phases dites d'amorçage et de rinçage. Lorsque cela se produit, il n'est pas toujours possible de retourner son sang au patient, lequel en perd alors une quantité non négligeable qui peut nécessiter une transfusion sanguine, surtout lorsque la situation se présente plusieurs fois en moins de 48 heures. Dans les cas cliniques les plus graves, la circulation extracorporelle est réalisée sous forme continue, définie comme étant de 24h/24 et sans interruption jusqu'à ce que la situation clinique du patient ne la nécessite plus, ce qui représente souvent plusieurs jours ou semaines. Un arrêt de la circulation extracorporelle du fait d'une coagulation du sang dans la boucle de circulation peut ainsi avoir différents effets négatifs selon la fonction assurée. Par exemple dans le cas de la dialyse, l'arrêt de la circulation du sang du fait de sa coagulation dans la boucle peut aboutir à une perte de sang tolérable en soi pour autant que cela soit rare ; dans ce cas, le traitement sera simplement poursuivi après avoir changé la boucle. Dans les cas de remplacement du coeur (CEC) et/ou des poumons (ECMO), un arrêt de la circulation peut mener à la mort du patient en quelques minutes si aucune intervention d'urgence n'est entreprise, laquelle est lourde et présente des risques d'échec. Dans tous les cas, un changement de boucle nécessite au moins 30 minutes, présente des risques pour le patient et coûte cher en matériel. Il est donc essentiel que la boucle puisse fonctionner sans interruption y compris en cas de coagulation du sang.

En pratique il est fréquent que le sang coagule dans les boucles de circulation extracorporelle ce qui se produit en quelques secondes voire 1 ou 2 minutes. Il est ainsi habituel d'avoir des petits caillots que l'on retrouvera surtout dans le filtre à caillots placé en sortie de la boucle et prévu pour les récupérer. Cependant comme la croissance des caillots est par nature exponentielle, ils peuvent grossir et prendre une ampleur suffisante pour fortement réduire voire bloquer la circulation dans la boucle en un point quelconque, le plus souvent dans l'élément d'épuration ou le filtre à caillot.

Pour prévenir cela, il est connu d'utiliser, à titre préventif, des substances définies comme étant anticoagulantes ; différents moyens largement décrits sont ainsi utilisés quotidiennement, principalement l'injection d'héparine ou de citrate dans la boucle de sang. Lorsque c'est nécessaire et possible, notamment du fait du faible taux de plaquettes dans le sang, la circulation du sang dans la boucle peut aussi être réalisée sans une telle anticoagulation préventive.

L'héparine, qui agit en activant l'antithrombine III, est généralement utilisée en anticoagulation systémique, c'est-à-dire que le sang du patient/donneur qui est anticoagulé dans la boucle le reste lorsqu'il retourne au patient, ce qui entraine, entre autres, des risques hémorragiques pour ce dernier. L'héparine est ainsi souvent déconseillée pour les patients ayant subi une opération chirurgicale, y compris une simple biopsie, dans les 24 heures précédents le traitement extracorporel.

Le citrate se lie lui au calcium ionisé (Ca⁺⁺), lequel est un élément incontournable de la cascade de coagulation sans lequel celle-ci ne peut se produire. Il a ainsi été démontré dans la publication de Calatzis et AI., « Citrate anticoagulation for extracorporeal circuits : Effects on whole blood coagulation activation and clot formation » ; Nephron 2001 ;89:233-236 qu'une concentration de calcium ionisé (Ca⁺⁺) dans le sang inférieure à 0.4 mmol/L de sang augmente le temps de coagulation, la courbe ayant une forme exponentielle et le sang ne coagulant plus à partir d'une concentration de calcium ionisé (Ca⁺⁺) dans le sang inférieure à 0.2 mmol/L. Ainsi l'injection de citrate dans le sang circulant dans la boucle extracorporelle a été mise en pratique clinique et largement décrite dans la littérature car elle présente sur l'héparine l'intérêt d'agir uniquement dans la boucle ; en effet après quelques centimètres parcourus dans la veine du patient, le sang issu de la boucle retrouve, en se mélangeant au sang du patient qui a lui des taux de 0.9-1.1 mmol/L, une concentration de calcium ionisé supérieure à 0.5 mmol/L, ce qui suspend immédiatement l'effet anticoagulant du citrate. On parle alors d'anticoagulation régionale puisqu'elle est limitée à la boucle de circulation extracorporelle, ce qui permet d'éviter les risques hémorragiques pour le patient et de prolonger le temps d'utilisation de la boucle de circulation extracorporelle tout au moins dans les cas les plus fréquents où le citrate est bien toléré par le patient.

Une autre technique permettant de réduire la coagulation dans la boucle consiste à diluer le sang, et donc la concentration de plaquettes, ce qui est obtenu en injectant au départ de la boucle une solution physiologique cristalline, appelée prédilution, méthode qui pour être efficace nécessite des quantités importantes de solution qu'il y a lieu de retirer avant le retour du sang au patient afin de ne pas le charger en fluide. Cette méthode ne s'applique donc qu'à l'hémofiltration et vient le plus souvent en complément à celles décrites ci-dessus. Elle présente de plus l'inconvénient de réduire l'efficacité du traitement puisque le sang traité est dilué et qu'il contient ainsi moins d'éléments à retirer.

Dans les trois cas ci-dessus de traitement d'anticoagulation préventive, la quantité injectée de solution visant à prévenir la coagulation résulte d'un optimum. Pour l'héparine, l'équilibre entre anticoagulation dans la boucle et risque de saignements du patient doit être trouvé. Le citrate qui se fixe à des ions essentiels à la vie comme le Ca⁺⁺ peut induire des déséquilibres menant à des situations cliniques catastrophiques voire à la mort puisque le sang retourné au patient possède une concentration en calcium ionisé de 0.2 à 0.4 mmol/L alors qu'une valeur inférieure à 0.6 mmol/L est létale. Selon les situations, la dose cumulée de citrate peut progressivement en effet faire baisser la concentration en calcium ionisé du patient et l'amener en-dessous de 0.6 mmol/L. La prédilution doit quant à elle être optimisée afin d'éviter une dilution du sang propre à réduire les capacités du traitement en-dessous du seuil désirable.

Ainsi le dosage des fluides injectés pour l'anticoagulation préventive est le fruit d'un équilibre délicat et empirique qui de plus fluctue sur la période de circulation du sang dans la boucle. Il est ainsi fréquent que l'anticoagulation préventive du sang dans le circuit soit insuffisante et que le sang coagule malgré tout, empêchant ainsi la circulation dans la boucle mais aussi que l'anticoagulation soit trop importante et devienne source de risques pour le patient. C'est d'autant plus vrai que le choix des débits des solutions d'anticoagulation préventive est de la responsabilité du personnel soignant lequel l'adapte en fonction de l'évolution de la situation tant celle existant dans le boucle que de l'état clinique du patient, les deux évoluant souvent de manière rapide, c'est à dire en quelques minutes. Une difficulté pratique supplémentaire provient du fait qu'il existe un délai de réponse entre les corrections de débits d'injection et le résultat de ces corrections qui ne peut être vérifié qu'après un temps qui se mesure en minutes voire en heures soit un temps durant lequel le personnel soignant effectuera de nombreuses autres tâches et laissera passer un délai qui entraîne des risques supplémentaires de coagulation dans la boucle. De plus, des paramètres du système varient de manière aléatoire, comme le débit du sang effectif, la présence d'air dans le circuit, les arrêts de la pompe à sang du fait d'alarmes ou l'hémoconcentration qui influent tous fortement, sans pouvoir être anticipés, sur la possible naissance de caillots dans la boucle. Il est ainsi impossible, sauf à injecter préventivement des doses massives et mal tolérées d'anticoagulant, d'éviter dans tous les cas une coagulation dans la boucle, ce que la pratique confirme quotidiennement.

Il est de plus connu que pour les différentes formes d'héparine les meilleures méthodes d'évaluation de l'anticoagulation produite, qui sont les mesures du temps de coagulation réalisées par des appareils conçus pour fournir des données normées, ne sont pas prédictives avec une fiabilité de 100% d'une coagulation de sang dans la boucle de circulation. De même pour le citrate, le sang coagule parfois du fait des limites imposées par la tolérance du patient à l'injection de citrate et à leurs variations dans le temps. Dans tous les cas il est donc possible que le sang présent dans la boucle de circulation coagule, malgré les moyens de prévention mis en oeuvre, phénomène progressif qui peut prendre de quelques minutes à quelques dizaines de minutes. Ainsi quelle que soit la substance utilisée en anticoagulation préventive le manque d'efficacité est en grande partie dû à la nécessité de minimiser la quantité de substance ce qui entraine naturellement le risque d'avoir une coagulation de sang dans le circuit, phénomène qui est observé de manière quotidienne dans les unités de soins et entraine soit l'arrêt du traitement en cours, avec éventuellement sa reprise après l'installation d'une nouvelle boucle, soit l'arrêt du traitement associé à une perte de sang du patient souvent égale au volume de la boucle de circulation extracorporelle (entre 200 ml et 2 litres) de sang ou encore des complications majeures du fait de l'arrêt de la circulation centrale et/ou de l'oxygénation du sang du patient.

Il apparait donc qu'il serait intéressant de pouvoir liquéfier, c'est-à-dire dissoudre le sang coagulé (solidifié) dans une boucle de manière à le rendre à nouveau fluide et éviter les problèmes décrits ci-dessus. La littérature parle de « fibrinolyse » qui est décrite comme une liquéfaction enzymatique des clous de fibrine et de « thrombolyse », ou thérapie par fibrinolyse, qui consiste à liquéfier des caillots présents dans les vaisseaux sanguins. Des systèmes de liquéfaction de caillots ont été décrits et sont utilisés pour des analyses de laboratoire ou à la suite d'attaques cérébrales pour déboucher des vaisseaux et ainsi rétablir le flux sanguin dans le patient par une méthode chimique ou au moyen d'une onde sonore. La fibrinolyse est ainsi documentée dans le cadre de pathologies impliquant la formation de caillots dans le corps comme les syndromes coronariens, les embolies pulmonaires ou l'infarctus du myocarde. En effet, la coagulation indésirable observée dans les boucles de circulation extracorporelle peut aussi se produire dans le corps humain et de la même manière bloquer la circulation sanguine. Un système naturel de fibrinolyse existe qui dissout les caillots et prévient habituellement, entre autres, les pathologies susmentionnées. Lorsque ce système ne fonctionne plus de manière satisfaisante, des médicaments peuvent être utilisés pour liquéfier les caillots ; il en existe principalement trois familles qui diffèrent par leur mode d'action soit les activateurs tissulaires du plasminogène, la streptokinase et l'urokinase. D'autres substances médicamenteuses existent mais la plupart ne sont efficaces que dans les premières heures des symptômes, démontrant ainsi que si la formation de caillots est réversible, elle évolue au cours du temps et en pratique devient de plus en plus irréversible.

Si la thrombolyse est surtout étudiée pour les pathologies susmentionnées, elle l'est aussi pour les abords vasculaires utilisés en dialyse. En effet, pour disposer d'un débit de sang suffisant à une circulation extracorporelle dans une boucle, il faut préparer un accès approprié, l'abord vasculaire, auquel on viendra connecter les entrées et sorties de la boucle. Ces accès sont principalement au nombre de trois, les fistules, les cathéters et les implants ; ils sont installés dans le patient au moyen d'une intervention chirurgicale. De manière fréquente (0.5-2 fois par an pour les implants, 0.1-0.5 fois par an pour les fistules), ils se retrouvent bouchés par des caillots. L'article de Quencer et Al., Hemodialysis access thrombosis, review article, Cardiovasc Diagn Ther 2017;S299-S308 présente les différentes solutions, et leurs limites, permettant de retirer les caillots des fistules et implants ainsi que la façon d'établir le diagnostic. L'article de Kennard et AI., Interventions for treating central venous haemodialysis cathéter malfunction (Review), Cochrane database of systematic reviews 2017, Issue 10. Art. No.:CD011953 discute les différentes études réalisées pour traiter des cathéters bouchés par des caillots. A la lecture de ces article, il apparait que plusieurs techniques de thrombolyse sont possibles avec leurs avantages et inconvénients respectifs mais également qu'il n'est pas démontré que l'une soit systématiquement supérieure aux autres.

Les substances qui peuvent être utilisées pour dissoudre les caillots présents agissent de différentes manières et à différents moments de la cascade de coagulation en visant directement les plaquettes, la fibrine ou les interactions entre les éléments constitutifs de la cascade par exemple en réduisant la concentration de Ca⁺⁺. Elles sont donc efficaces à des instants différents du processus de coagulation lequel possède de nombreuses étapes et chemins possibles. Par nature les cas habituellement étudiés et décrits ci-dessus s'adressent à des caillots formés depuis plusieurs heures voire quelques jours. Les moyens utilisés et les résultats obtenus dans ces cas peuvent ainsi être très différents de ceux que l'on rencontre dans une boucle de sang où l'on se situe dans les premières secondes ou minutes de la formation d'un caillot.

Les boucles de circulation extracorporelle de sang possèdent toujours des mesures de pressions utilisées pour déterminer si la circulation du sang forcée par une pompe se fait comme requis. Il est ainsi possible, et connu, de détecter une coagulation naissante dans une boucle, au moyen par exemple de la mesure des pressions en amont et en aval d'un élément comme un dialyseur ou la pression en amont d'un filtre à caillot placé pour retenir lesdits caillots avant la réinjection du sang au patient. En effet, le sang coagulé ne circule pas, ou très peu, et bouche au moins partiellement le conduit dans lequel il se trouve ; il va donc freiner la circulation du sang et si une pompe est utilisée pour forcer le débit la différence de pression entre la partie qui se trouve en amont et celle qui est en aval de sa position va augmenter. Lorsque cela se produit, les plaquettes se collant les unes aux autres, il existe un effet « boule de neige » qui fait que le phénomène s'accélère rapidement. Dans de tels cas, en pratique courante il n'existe pas de moyens de poursuivre le traitement au-delà de quelques minutes après que la présence de caillots soit avérée et il n'est même pas toujours possible de retourner son sang au patient/donneur avant que le circuit soit totalement bouché.

Les systèmes d'anticoagulation utilisés pour les boucles de circulation extracorporelle et décrits dans la littérature se concentrent tous sur la prévention de la coagulation en injectant notamment de l'héparine ou du citrate selon des modèles qui varient en complexité et dont l'adéquation doit être réévaluée soit par le personnel soignant soit automatiquement au moins une fois par heure. Lorsque des caillots se créent dans la boucle, il serait cependant intéressant de procéder à une liquéfaction desdits caillots afin de pouvoir poursuivre la circulation du sang et les traitements y relatifs plutôt que de changer la boucle comme cela se fait habituellement. Cependant les méthodes décrites ci-dessus et utilisées en laboratoire ou dans le corps du patient ne sont pas applicables à une boucle de circulation de sang car elles sont soit trop lentes soit trop compliquées. De facto, une boucle extracorporelle de sang est particulière du point de vue de la formation de caillots puisque le sang est d'une part fortement activé et, pour contrebalancer cela, fortement anticoagulé. De plus, dans ce cas il est possible de détecter rapidement la formation de caillots et donc d'agir à un stade précoce de la cascade de coagulation.

Par exemple la demande de brevet EP 1095666 décrit un système s'appliquant au traitement dit d'hémofiltration qui mesure des paramètres représentant l'écoulement du sang et de l'ultrafiltrat, qui est extrait du sang à travers un filtre, et adapte en conséquence le débit de prédilution, qui consiste en une solution physiologique apportée en amont du filtre, dans le but principal de supprimer l'encrassement dans le filtre tout en ajustant automatiquement le volume d'échange. Ce document décrit les moyens possibles pour déterminer l'état de circulation dans une boucle. Son application est cependant limitée à une boucle d'hémofiltration qui est nécessaire pour retirer la prédilution. En effet, le rinçage d'une boucle par prédilution requiert l'injection de quantités importantes de fluide, typiquement à un débit d'au moins 1 l/h, qui ne sauraient être accumulées dans le patient et doivent donc être éliminées. De plus, la prédilution possède essentiellement des propriétés de décrassage du filtre par réduction de la viscosité sanguine et augmentation du débit sanguin ; elle réduit un peu la coagulation en diluant le sang, donc la concentration de plaquettes dont la probabilité de se rencontrer diminue ainsi, mais cet effet est souvent insuffisant à prévenir la formation de caillots. Cette invention présente l'intérêt de mesurer les conditions de circulation dans une boucle utilisée en hémofiltration, et leurs variations, pour adapter le débit d'une substance, la prédilution, améliorant cette circulation. Elle n'a cependant aucun pouvoir de liquéfaction de caillots existants et nécessite l'apport de quantités d'eau importantes qui doivent être retirées de la boucle avant de retourner le sang au patient. Elle est donc spécifique à un type de traitement, l'hémofiltration, qui permet d'extraire par filtration de larges quantités d'eau.

Un autre exemple d'art antérieur est US2012/273415 A1. Ce document explique qu'une des mesures correctives qu'il est possible de prendre pour empêcher qu'une membrane ne s'encrasse davantage est d'augmenter la concentration en anticoagulant, et qu'alternativement ou en parallèle, il est également possible d'ajouter un agent thrombolytique pour nettoyer ou décrasser la membrane. Une fois la membrane dégagée, la concentration en anticoagulant peut être réduite et l'utilisation de l'agent thrombolytique peut être arrêtée.

Il a été observé par l'inventeur qu'ajouter du citrate comme solution de liquéfaction avec une concentration au moins double de celle utilisée en prévention permet de liquéfier les caillots pour autant que l'on intervienne dès les premières minutes où le phénomène prend de l'ampleur, alors qu'ajouter de l'héparine lorsque le sang commence à coaguler dans une boucle est parfaitement inutile de même que de faire circuler du sérum physiologique à la place du sang. Ces ajouts sont toujours faits par un opérateur et basés sur l'appréciation personnelle et souvent tardive de l'intervenant. Il en résulte souvent la nécessité de devoir changer la boucle qui est alors bouchée par des caillots.

Le but de la présente invention est de remédier, au moins en partie, aux inconvénients susmentionnés.

A cet effet, la présente invention concerne un dispositif de circulation extracorporelle du sang ayant les caractéristiques techniques de la revendication 1, ce dispositif comportant une boucle de circulation du sang qui comprend au moins un conduit d'extraction du sang et un conduit de retour du sang, ainsi que des moyens agencés pour faire circuler le sang dans la boucle de circulation, et une unité de commande comprenant des moyens de calcul agencés pour déterminer au moins une valeur de seuil dudit paramètre, l'enregistrer dans une mémoire, et comparer ensuite la valeur courante dudit paramètre avec ladite valeur de seuil enregistrée.

Selon l'invention, ledit dispositif comprend également au moins une source de solution de liquéfaction de caillots sanguins, des moyens d'injection et un ou plusieurs conduit(s) de liaison permettant d'acheminer ladite solution de liquéfaction dans la boucle de circulation. De plus, l'unité de commande comprend des moyens de pilotage d'au moins un desdits moyens d'injection de ladite solution de liquéfaction, et ladite unité de commande est agencée pour actionner lesdits moyens de pilotage lorsque ladite valeur de seuil est dépassée, signifiant la formation d'au moins un caillot, lesdits moyens de pilotage étant agencés pour que la solution de liquéfaction soit présente dans la boucle de circulation avec une quantité et pour une durée prédéterminées comme étant nécessaires et au moins suffisantes pour permettre la liquéfaction du caillot formé.

Lesdits moyens de pilotage agiront en fonction de paramètres tels qu'un débit, un temps ou un volume, l'essentiel étant que la quantité de liquide de liquéfaction, déterminée pour le volume de sang présent dans la boucle, et le temps, déterminé par le mode d'action de la solution de liquéfaction, soit suffisants pour liquéfier les caillots présents dans la boucle. A l'évidence pour l'homme de l'art, lesdits moyens de pilotage agiront en actionnant les moyens de circulation de la solution de liquéfaction et/ou les moyens de circulation du sang de manière à assurer que la quantité et le temps sont être respectés en tous points de la boucle.

Ainsi les moyens de pilotage peuvent être également agencés pour commander les moyens agencés pour faire circuler le sang dans la boucle de circulation selon différents scénarios, par exemple en réduisant le débit de sang dans la boucle afin de laisser plus de temps pour que la solution de liquéfaction agisse ou en arrêtant complétement le sang pour une durée déterminée une fois que la boucle a été remplie d'une quantité, fournissant une concentration, estimée comme suffisante de solution de liquéfaction. De nombreuses autres possibilités de pilotage connues de l'homme de l'art existent, par exemple celle qui consiste à reproduire à intervalle de temps réguliers, ou après un événement particulier comme un arrêt des moyens de circulation du sang, une injection de solution de liquéfaction de manière à liquéfier tout caillot naissant. Les différents scénarios ne sont en aucun cas exclusifs et peuvent en pratique être combinés pour déterminer le pilotage des moyens de circulation du liquide de liquéfaction.

Le dispositif selon l'invention comprend de plus dans la boucle de circulation des moyens de mesure d'au moins un des paramètres influencés par la résistance à l'écoulement du sang, puisque ladite résistance est modifiée par la présence d'éventuels caillots dans le boucle de circulation. Ces moyens peuvent être agencés pour mesurer une pression ou un débit.

Les moyens de calcul sont agencés pour comparer une valeur courante dudit paramètre avec ladite valeur de seuil enregistrée à une fréquence suffisante pour permettre la liquéfaction du caillot en formation soit préférentiellement une fois par seconde mais au moins une fois par minute.

De préférence, la solution de liquéfaction est composée d'eau et d'un élément choisi parmi un groupe comprenant le citrate, l'urokinase, la streptokinase, et leurs mélanges présents dans une quantité suffisante pour permettre la liquéfaction d'un caillot. Toute autre substance ou mélange connu ou à venir peut bien sûr être utilisé dans la solution de liquéfaction pour autant que ses effets sur les caillots et ceux indésirables sur le patient soient déterminés.

D'une manière avantageuse, le dispositif de l'invention peut aussi comprendre des moyens agencés pour réaliser une anticoagulation préventive.

Ainsi, la présente invention propose un dispositif ou appareil qui possède une boucle de circulation du sang destinée à un usage quelconque (dialyse, CEC, aphérèse, ECMO) comme décrit précédemment et qui permette premièrement de détecter que du sang est en cours de coagulation et deuxièmement de réaliser une liquéfaction des caillots en injectant une substance dont une faible quantité, idéalement inférieure à 100 ml, est suffisante à atteindre ce but. Elle permet donc de liquéfier le sang qui a coagulé (c'est-à-dire le sang qui, de l'état liquide, s'était transformé en solide pour former un caillot dans la boucle) et ainsi de poursuivre la circulation du sang sans avoir à changer la boucle.

D'autres caractéristiques et avantages de la présente invention apparaîtront à la lecture de la description détaillée suivante de plusieurs modes de réalisation de l'invention, donnés à titre d'exemples non limitatifs, et faite en référence aux dessins annexés dans lesquels :
- la figure 1 représente une vue schématisée d'un dispositif selon l'invention,
- la figure 2 est un schéma bloc de l'unité de commande ; et
- la figure 3 illustre une suite d'opérations de fonctionnement de l'unité de commande selon l'invention.

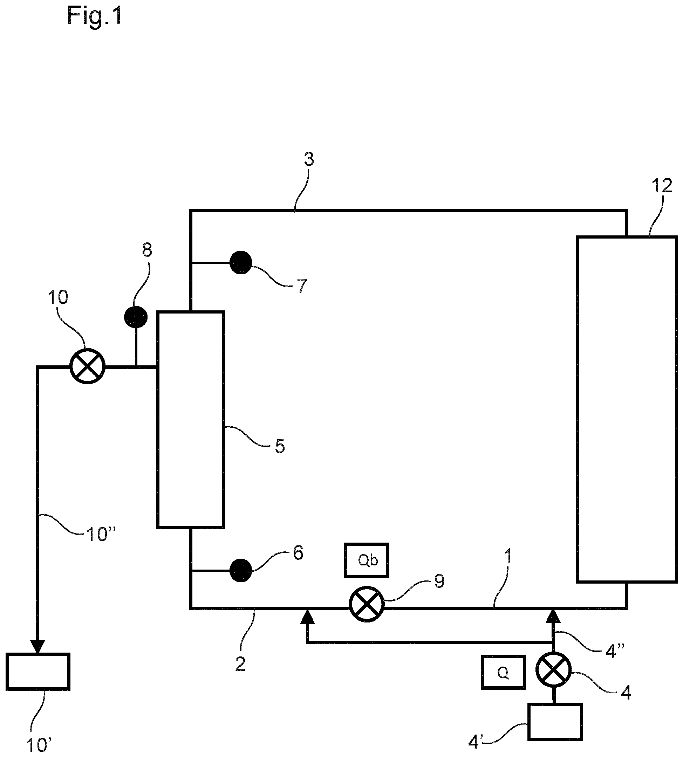

Un appareil selon la présente invention possède, comme représenté sur la figure 1, une boucle de circulation extracorporelle du sang comprenant au moins un conduit d'extraction du sang comprenant un conduit 1 d'extraction du sang du patient ou donneur et un conduit de retour du sang 3. Ladite boucle peut être complétée par un conduit 2 et des moyens de modification du sang 5. L'appareil comprend également des moyens 9 agencés pour faire circuler le sang dans la boucle de circulation. Sur la boucle de circulation sont placés des moyens d'injection 4 permettant d'injecter un fluide contenant une solution 4' apte à liquéfier des caillots de sang dans le conduit 1 et/ou le conduit 2; cette solution 4' peut être contenue dans un réservoir quelconque comme une poche, une seringue ou une bouteille.. Lesdits moyens d'injection comprennent un conduit de liaison 4" et des moyens agencés pour faire circuler la solution de liquéfaction 4' dans ledit conduit de liaison 4", telle qu'une pompe 4.

Le dispositif peut également comprendre des moyens de modification du sang 5, constitués par exemple de filtres, dialyseurs, cartouches d'adsorption ou d'un oxygénateur. Ces moyens 5, qui ne sont pas présents dans le cas d'une pure CEC, peuvent en pratique être composés de plusieurs éléments comme par exemple d'un dialyseur et d'une cartouche d'adsorption. L'appareil peut posséder de plus des moyens de mesure 6, 7, 8 d'au moins un des paramètres influencés par la résistance à l'écoulement du sang dans la boucle de circulation. Ces moyens de mesure peuvent être des capteurs de pression ou des capteurs débits 6 et/ou 7 et/ou 8 qui sont habituellement utilisés pour s'assurer que la circulation dans la boucle est conforme aux attentes et notamment qu'il n'y a ni déconnection ni plicature de conduit. Pour la dialyse et l'aphérèse, l'appareil peut être complété par des moyens d'extraction du fluide obtenu à partir des moyens de modification du sang 5, lesdits moyens d'extraction comprenant une pompe 10, un conduit 10", et des moyens de rejet 10'. La boucle de circulation du sang est de plus complétée en pratique par les moyens habituels mais non représentés de protection du patient ou donneur 12, comme par exemple un détecteur de fuite de sang ou un détecteur d'air associé à un clamp et d'autres moyens d'injection de solutions comme par exemple une pompe à seringue utilisée pour l'anticoagulation préventive ou des moyens de substitution du fluide extrait. Ces moyens non représentés sont largement connus de l'homme de l'art et décrits dans la littérature, ils peuvent bien sûr faire partie de l'appareil décrit dans la présente invention.

Les moyens de circulation des fluides 4, 9, 10, utilisés pour la présente invention telle que représentée par le circuit de la figure 1 peuvent en pratique être indifféremment des pompes péristaltiques, des pompes à membranes, des pompes centrifuges, des pousse-seringue ou des clamps, éléments bien connus et utilisés dans les applications nécessitant une boucle de circulation extracorporelle du sang. Ces moyens sont complétés de réservoirs, de conduits et de raccords.

Les éléments indispensables pour la présente invention sont la boucle de circulation de sang composée des conduits 1 et 3, des moyens de circulation du sang 9, des moyens d'injection d'une solution 4, 4', 4" et une unité de commande comme décrite ci-après. Les autres éléments sont facultatifs pour un appareil selon l'invention et ajoutés ou pas en fonction de l'utilisation prévue de la boucle de sang.

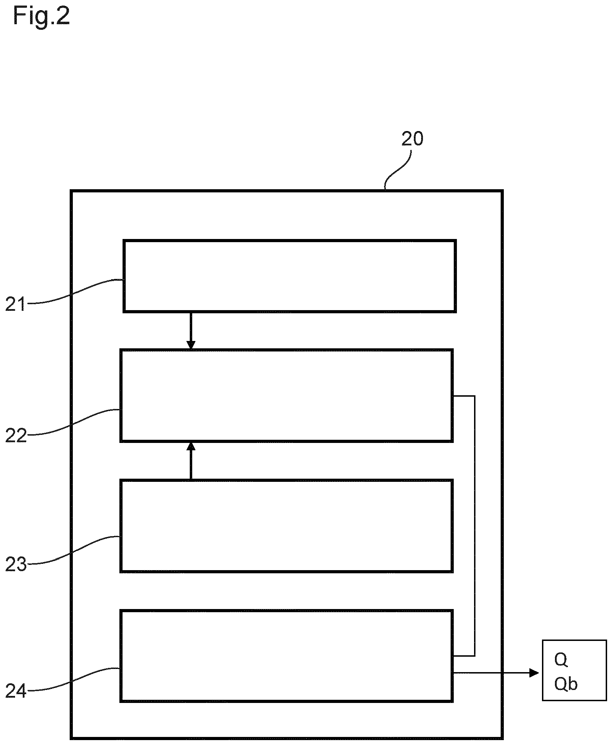

L'appareil selon l'invention possède ainsi une unité de commande 20, telle qu'elle est représentée sur la figure 2, composée d'une interface utilisateur 21 qui permet l'affichage des messages nécessaires et le cas échéant la saisie des valeurs déterminantes de l'invention. Les moyens de calcul 22 agissent en fonction des règles préétablies pour liquéfier les caillots et envoie à l'interface utilisateur les messages nécessaires en prenant lorsque cela est requis des informations qui sont enregistrées dans la mémoire 23. Les règles préétablies font appel à des équations connues mais peuvent aussi intégrer des conditions nouvelles, notamment des limites hautes et basses fournies via l'interface utilisateur 21. Parmi les équations connues on peut citer le fait qu'au liquide de liquéfaction 4' est associé un débit « Q » représenté par la pompe 4. La quantité injectée « M » par unité de temps « t » est donnée par le produit du débit « Q » avec le temps correspondant soit : M = Q * t alors que le temps d'injection « T » peut être calculé connaissant le volume de sang « V » sur lequel on veut agir, par exemple 2 x le volume compris dans la boucle, avec : T - V/Qb, où Qb est le débit de sang donné par la pompe 9. Les moyens de pilotage 24 de l'unité de commande commanderont alors les pompes 4 et 9 en fonction des mesures et calculs effectués.

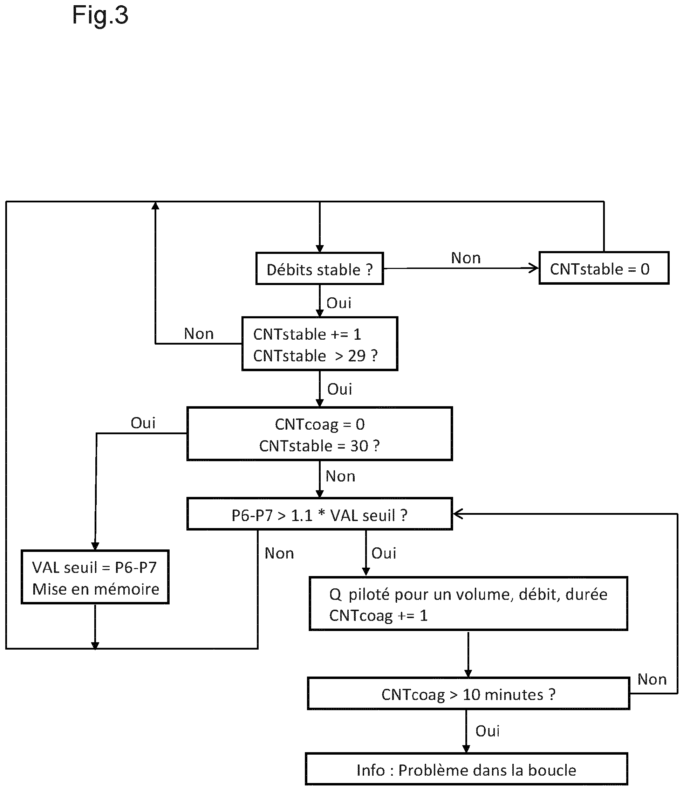

L'unité de calcul d'un appareil selon l'invention peut par exemple fonctionner selon un algorithme tel qu'illustré sur la figure 3. En régime stable de débits, déterminé par exemple pour une vitesse constante de la pompe à sang 9 pendant 30 secondes, l'unité de commande 20 enregistre la différence entre la pression 6 et la pression 7 dans la mémoire 23, différence qui est remise à jour à chaque fois que les conditions de stabilité disparaissent, ce qui initialise le compteur de durée stable. Lorsque la différence mesurée augmente par rapport à celle enregistrée, par exemple de plus de 10%, l'unité de commande 20 pilote la pompe 4 de solution de liquéfaction 4' à un débit et pour une durée prédéterminés pour dissoudre les caillots en formation, puis elle vérifie que la différence revient en-dessous des 10% souhaités dans les 10 minutes qui suivent. Dans le cas contraire, l'unité de commande 20 informe l'utilisateur, via l'interface 21 qu'il y a un problème dans la boucle. D'autres algorithmes sont bien sûr possibles, l'essentiel étant qu'ils procèdent en déterminant premièrement d'une manière quelconque que des caillots sont en train de se former dans la boucle, typiquement en utilisant des pressions ou débits mesurés par 6, 7 ou 8 et en appliquant des règles comme par exemple celles décrites dans la demande de brevet EP 1095666, puis en injectant pendant une durée et à un débit déterminés une solution propre à liquéfier les caillots existants. Durée et débits peuvent bien sûr être remplacés par d'autres paramètres comme par exemple un volume injecté jusqu'à ce que la différence entre les pressions 6 et 7 soit à maximum 5% au-dessus de celle enregistrée mais au plus de 100 ml. Les moyens 6, 7 ou 8 peuvent être remplacés ou complétés par des intervalles de temps ou des situations prédéterminés comme par exemple un arrêt prolongé au-delà de 2 minutes des moyens de circulation du sang lesquels sont connus pour être souvent à l'origine de la formation de caillots.

Pour illustrer ceci, on peut prendre l'exemple d'une aphérèse réalisée avec une anticoagulation préventive qui injecte en permanence à un débit égal à 5% du débit sang de 150 ml/min, un fluide contenant une solution 4' avec 4% de citrate , la pompe 4 fournissant ainsi un débit 7.5 ml/min. La différence de pressions 6-7 indique au départ 60 mmHg. Lorsque cette différence est supérieure à 66 mmHg, la pompe 4 double son débit pour injecter non plus 5% mais 10% de citrate par rapport au débit sanguin, ceci pendant 3 minutes puis la pompe revient à son débit initial et l'unité de commande 20 contrôle que la différence 6-7 est désormais inférieure à 63 mmHg. Dans la même application, le pilotage des pompes pourrait être réalisé non pas en doublant la vitesse de la pompe 4 mais en diminuant celle de la pompe 9 de moitié. Cette solution présenterait l'avantage de ne pas augmenter la dose de citrate injectée au patient tout en fournissant la même concentration de citrate dans le sang et donc le même effet de liquéfaction.

La circulation de citrate se fait sur 1-2 volume(s) de la boucle soit dans un exemple d'aphérèse ou de dialyse sur 200-400 ml ce qui représente une pulse entre 16 et 40 ml de solution, soit un volume faible par rapport à l'anticoagulation préventive. Cette pulse appliquée au bon moment permet au traitement de se poursuivre tant que cela est cliniquement indiqué, et de réduire ainsi les pertes de sang du patient et les risques de transfusion associés. On constate par cet exemple que les moyens 4, 4' et 4" prévus pour injecter la solution de liquéfaction peuvent aussi être utilisés pour l'anticoagulation préventive ce qui a l'avantage de réduire le matériel nécessaire ainsi que le temps nécessaire à son installation.

Si l'anticoagulation préventive, c'est-à-dire celle visant à prévenir la formation de caillots dans la boucle, est faite avec un autre moyen qu'un soluté au citrate comme dans l'exemple ci-dessus, la situation serait la même et une pulse de citrate produirait le même effet.

De nombreuses variantes peuvent être utilisées pour liquéfier des caillots contenus dans une circulation extracorporelle ; toutes consistent à injecter de la solution liquéfiante à un moment donné et pour une durée et un volume déterminés comme étant nécessaires et suffisants. Il est optimal que ledit moment donné soit déterminé en se basant sur la détection de la formation des caillots avec des moyens de mesure de pression ou de débits 6, 7 ou 8 car cela permet d'agir à chaque fois, et uniquement lorsque, un caillot est en formation. Une variante possible serait cependant d'injecter la solution liquéfiante à intervalle de temps prédéterminés, par exemple toutes les 20 minutes, ou sur la base d'événements, par exemple un arrêt de la pompe à sang de plus de 2 minutes, mais cela ne serait pas optimal et présenterait le risque d'une injection trop tardive aboutissant à un résultat identique à celui de l'anticoagulation préventive.

Ainsi, un autre exemple serait celui d'une dialyse réalisée avec une anticoagulation préventive à l'héparine, laquelle serait injectée par un pousse-seringue (non représenté). Pour satisfaire à l'invention, l'appareil de dialyse comporterait de plus des moyens d'injection 4, 4', 4" d'une solution contenant 4% de citrate qui serait pilotée comme décrit dans le cas précédent, ou une solution contenant de l'urokinase ou un mélange des deux qui serait injectée pendant 90 secondes de manière à ce que tout le sang contenu dans la boucle soit mélangé à la solution de liquéfaction avant d'arrêter les pompes 4 et 9 pendant 3 minutes afin de laisser le temps à la solution d'agir et liquéfier les caillots. En variante, plutôt que de mesurer uniquement la différence 6-7, l'unité de commande 20 pourrait mesurer de plus la pression transmembranaire (PTM) définie par ((6+7)/2) -8) et piloter la pompe 4 non seulement sur la base de la différence 6-7 mais aussi sur celle d'une augmentation de la PTM.

Un troisième exemple serait une CEC ne comportant pas d'anticoagulation préventive. La pompe 4 pourrait dans ce cas être un pousse-seringue dont la seringue de 60 ml serait remplie d'une solution comportant un mélange de citrate à 20% et de streptokinase. Lorsque la pression de retour du sang 7 augmente indiquant la présence d'un caillot en aval, un bolus de 10 ml est injecté par le pousse-seringue qui peut ainsi liquéfier successivement 6 caillots avant d'informer l'opérateur de la nécessité de remplir la seringue.

Un avantage essentiel de la présente invention réside dans le fait qu'elle permet, par rapport aux techniques existantes, de réduire significativement les risques liés à l'usage des anticoagulants puisque ceux-ci peuvent être dosés « a minima », les éventuels caillots étant détectés et liquéfiés automatiquement en utilisant une faible quantité de solution de liquéfaction puisqu'elle est ponctuelle et ne s'adresse qu'au volume de sang contenu dans la boucle (qui est typiquement de 200 ml en aphérèse et dialyse) et non au volume de sang cumulé (typiquement 200 ml/min de débit sang soit 12 l/h) ou à celui du patient (5 litres environ pour un adulte de 70 kg). L'invention permet ainsi non seulement d'utiliser un minimum d'anticoagulants mais aussi d'éviter les inconvénients d'une boucle contenant du sang coagulé en proposant des moyens permettant de liquéfier les caillots.

Les variantes de réalisation selon l'invention s'appliquent à toutes les différentes configurations de boucles de circulation extracorporelle, elles intègrent la possibilité d'utiliser les différentes solutions possibles, comprenant une ou plusieurs substance(s) à même de liquéfier les caillots, et de les injecter en différents endroits du circuit mais de préférence avant la pompe à sang 9.

## Revendications

1. Dispositif de circulation extracorporelle du sang comportant une boucle de circulation du sang comprenant au moins un conduit d'extraction du sang (1) et un conduit de retour du sang (3), des moyens (9) agencés pour faire circuler le sang dans la boucle de circulation, des moyens de mesure d'au moins un paramètre influencé par la résistance à l'écoulement du sang dans la boucle de circulation, et ledit dispositif comportant une source de solution de liquéfaction (4') de caillots sanguins, des moyens d'injection (4, 4") de ladite solution de liquéfaction (4') dans la boucle de circulation (1,3) et une unité de commande (20) comprenant des moyens de calcul (22) agencés pour déterminer au moins une valeur de seuil dudit paramètre et l'enregistrer en mémoire, et agencés en outre pour comparer ensuite la valeur courante dudit paramètre avec ladite valeur de seuil enregistrée, l'unité de commande (20) comprenant des moyens de pilotage (24) desdits moyens d'injection (4) de ladite solution de liquéfaction (4') dans la boucle de circulation (1,3), et l'unité de commande étant prévue pour actionner les moyens de pilotage (24) des moyens d'injection (4) lorsque ladite valeur de seuil est dépassée, **caractérisé en ce que** l'unité de commande (20) est prévue pour actionner lesdits moyens de pilotage (24) des moyens d'injection (4) de manière que la solution de liquéfaction (4') soit présente dans la boucle de circulation avec une quantité et pour une durée prédéterminées comme nécessaires et suffisantes pour permettre la liquéfaction du ou des caillot(s) formé(s) dans ladite boucle.

2. Dispositif selon la revendication 1 **caractérisé en ce que** l'unité de commande (20) est agencée pour déterminer ou recevoir de l'interface utilisateur (21) au moins un intervalle de temps, l'enregistrer dans une mémoire (23), et comparer la valeur courante du temps avec ledit intervalle pour actionner les moyens de pilotage (24) des moyens d'injection (4) agencés pour faire circuler la solution de liquéfaction (4') dans un conduit de liaison (4").

3. Dispositif selon l'une des revendications précédentes, **caractérisé en ce que** les moyens de calcul (22) sont agencés pour commander les moyens de pilotage (24), en réduisant ou arrêtant le débit d'une pompe à sang (9), de manière à ce que la solution de liquéfaction (4') soit présente pour une durée déterminée comme suffisante dans la boucle de circulation.

4. Dispositif selon l'une des revendications précédentes, **caractérisé en ce que** la boucle de circulation du sang est complétée par des moyens de modification du sang (5) et un conduit (2).

5. Dispositif selon l'une des revendications précédentes, **caractérisé en ce que** les moyens de mesure (6, 7, 8) d'au moins un des paramètres influencés par la résistance à l'écoulement du sang dans la boucle de circulation sont agencés pour mesurer une pression.

6. Dispositif selon l'une des revendications 1 à 4, **caractérisé en ce que** les moyens de mesure (6, 7, 8) d'au moins un des paramètres influencés par la résistance à l'écoulement du sang dans la boucle de circulation sont agencés pour mesurer un débit.

7. Dispositif selon l'une des revendications précédentes, **caractérisé en ce qu'**il comporte en outre des moyens pour effectuer une anticoagulation préventive.

8. Dispositif selon l'une des revendications précédentes, **caractérisé en ce que** les moyens d'injection (4), la solution de liquéfaction (4') et le conduit de liaison (4") sont utilisés pour effectuer une anticoagulation préventive.

9. Dispositif selon l'une des revendications précédentes, **caractérisé en ce que** la solution de liquéfaction (4') comprend un élément choisi parmi le groupe comprenant le citrate, l'urokinase, la streptokinase et leurs mélanges.

## Patentansprüche

1. Vorrichtung für die extrakorporale Blutzirkulation, die eine Blutzirkulationsschleife umfasst, die mindestens eine Blutextraktionsleitung (1) und eine Blutrückleitung (3), Mittel (9), die dazu gestaltet sind, das Blut in der Zirkulationsschleife zum Zirkulieren zu bringen, Mittel zur Messung mindestens eines Parameters, der durch den Strömungswiderstand des Blutes in der Zirkulationsschleife beeinflusst wird, umfasst und wobei die Vorrichtung eine Quelle für Verflüssigungslösung (4') von Blutgerinnseln, Mittel (4, 4") zur Einspritzung der Verflüssigungslösung (4') in die Zirkulationsschleife (1, 3) und eine Steuereinheit (20) umfasst, die Berechnungsmittel (22) umfasst, die dazu gestaltet sind, mindestens einen Schwellenwert des Parameters zu bestimmen und ihn im Speicher aufzuzeichnen, und ferner dazu gestaltet sind, dann den aktuellen Wert des Parameters mit dem aufgezeichneten Schwellenwert zu vergleichen, wobei die Steuereinheit (20) Mittel (24) zur Lenkung der Mittel (4) zur Einspritzung der Verflüssigungslösung (4') in die Zirkulationsschleife (1, 3) umfasst, und die Steuereinheit dazu vorgesehen ist, die Lenkungsmittel (24) der Einspritzmittel (4) zu betätigen, wenn der Schwellenwert überschritten wird, **dadurch gekennzeichnet, dass** die Steuereinheit (20) zum Betätigen der Mittel (24) zur Lenkung der Einspritzmittel (4) vorgesehen ist, derart dass die Verflüssigungslösung (4') in der Zirkulationsschleife mit einer vorbestimmten Menge und während einer vorbestimmten Dauer, wie zum Ermöglichen der Verflüssigung des/der in der Schleife gebildeten Blutgerinnsel/s erforderlich und ausreichend bestimmt, vorhanden ist.

2. Vorrichtung nach Anspruch 1, **dadurch gekennzeichnet, dass** die Steuereinheit (20) dazu gestaltet ist, mindestens ein Zeitintervall zu bestimmen oder von der Benutzeroberfläche (21) zu empfangen, es in einem Speicher (23) aufzuzeichnen und den aktuellen Wert der Zeit mit dem Intervall zu vergleichen, um die Mittel (24) zur Lenkung der Einspritzmittel (4) zu betätigen, die dazu gestaltet sind, die Verflüssigungslösung (4') in einer Verbindungsleitung (4") zum Zirkulieren zu bringen.

3. Vorrichtung nach einem der vorhergehenden Ansprüche, **dadurch gekennzeichnet, dass** die Berechnungsmittel (22) dazu gestaltet sind, die Lenkungsmittel (24) durch Vermindern oder Anhalten des Durchsatzes einer Blutpumpe (9) derart zu steuern, dass die Verflüssigungslösung (4') während einer als ausreichend bestimmten Dauer in der Zirkulationsschleife vorhanden ist.

4. Vorrichtung nach einem der vorhergehenden Ansprüche, **dadurch gekennzeichnet, dass** die Blutzirkulationsschleife durch Blutänderungsmittel (5) und eine Leitung (2) ergänzt ist.

5. Vorrichtung nach einem der vorhergehenden Ansprüche, **dadurch gekennzeichnet, dass** die Mittel (6, 7, 8) zur Messung von mindestens einem der Parameter, die durch den Strömungswiderstand des Blutes in der Zirkulationsschleife beeinflusst werden, dazu gestaltet sind, einen Druck zu messen.

6. Vorrichtung nach einem der Ansprüche 1 bis 4, **dadurch gekennzeichnet, dass** die Mittel (6, 7, 8) zur Messung mindestens eines der Parameter, die durch den Strömungswiderstand des Blutes in der Zirkulationsschleife beeinflusst werden, dazu gestaltet sind, einen Durchsatz zu messen.

7. Vorrichtung nach einem der vorhergehenden Ansprüche, **dadurch gekennzeichnet, dass** sie ferner Mittel zum Durchführen einer präventiven Antikoagulation umfasst.

8. Vorrichtung nach einem der vorhergehenden Ansprüche, **dadurch gekennzeichnet, dass** die Einspritzmittel (4), die Verflüssigungslösung (4') und die Verbindungsleitung (4") zum Durchführen einer präventiven Antikoagulation verwendet werden.

9. Verfahren nach einem der vorhergehenden Ansprüche, **dadurch gekennzeichnet, dass** die Verflüssigungslösung (4') ein Element umfasst, das ausgewählt wird aus der Gruppe, die Citrat, Urokinase, Streptokinase und ihre Gemische umfasst.

## Claims

1. Extracorporeal blood circulation device including a blood circulation circuit comprising at least one blood extraction line (1) and a blood return line (3), means (9) arranged to circulate the blood in the circulation circuit, means for measuring at least one parameter influenced by the resistance to flow of blood in the circulation circuit, and said device comprising a source of solution (4') for liquefying blood clots, means (4, 4") for injecting said liquefaction solution (4') into the circulation circuit (1, 3) and a control unit (20) comprising calculation means (22) arranged to determine at least one threshold value of said parameter and record it in a memory, and further arranged to then compare the current value of said parameter with said recorded threshold value, the control unit (20) comprising means (24) for driving said means (4) for injecting said liquefaction solution (4') into the circulation circuit (1, 3), and the control unit being provided to actuate said means (24) for driving the injection means (4) when said threshold value is exceeded, **characterised in that** the control unit (20) is provided to actuate said means (24) for driving said injection means (4) so that the liquefaction solution (4') is present in the circulation circuit in an amount and for a time which are predetermined to be necessary and sufficient for enabling the liquefaction of the clot(s) formed in said circuit.

2. Device according to claim 1, **characterised in that** the control unit (20) is arranged to determine or receive from the user interface (21) at least one time interval, record it in a memory (23), and compare the standard value of the time with said interval to actuate the means (24) for driving the injection means (4) arranged to circulate the liquefaction solution (4') in a connecting line (4").

3. Device according to one of the preceding claims, **characterised in that** the calculation means (22) are arranged to control the driving unit (24), by reducing or stopping the flow of a blood pump (9), so that the liquefaction solution (4') is present for a time considered as sufficient in the circulation circuit.

4. Device according to one of the preceding claims, **characterised in that** the blood circulation circuit is completed by blood modification means (5) and a line (2).

5. Device according to one of the preceding claims, **characterised in that** the means (6, 7, 8) for measuring at least one of the parameters influenced by the resistance to flow of blood in the circulation circuit are arranged to measure a pressure.

6. Device according to one of claims 1 to 4, **characterised in that** the means (6, 7, 8) for measuring at least one of the parameters influenced by the resistance to flow of blood in the circulation circuit are arranged to measure a flow rate.

7. Device according to one of the preceding claims, **characterised in that** it further includes means for performing preventive anticoagulation.

8. Device according to one of the preceding claims, **characterised in that** the injection means (4), the liquefaction solution (4') and the connecting line (4") are used for performing preventive anticoagulation.

9. Device according to one of the preceding claims, **characterised in that** the liquefaction solution (4') comprises a component chosen from the group comprising citrate, urokinase, streptokinase and mixtures thereof.
